# EUROPÄISCHE PATENTSCHRIFT

(11) **EP 2 265 186 B1**
(45) Veröffentlichungstag und Bekanntmachung des Hinweises auf die Patenterteilung: **16.11.2011**
(21) Anmeldenummer: 09733108.6
(22) Anmeldetag: 16.04.2009
(51) Int. Cl.: A61B 17/00, A61B 17/221

(54) **MEDIZINISCHER AUFNAHMEBEUTEL UND VERFAHREN ZU DESSEN HERSTELLUNG**
MEDICINAL RECEIVING BAG AND METHOD FOR THE PRODUCTION THEREOF
POCHE COLLECTRICE À USAGE MÉDICAL ET SON PROCÉDÉ DE FABRICATION

(30) Priorität: 17.04.2008 DE 102008019497
(43) Veröffentlichungstag der Anmeldung: 29.12.2010
(73) Patentinhaber: Medi-Globe GmbH, 83101 Rohrdorf-Achenmühle (DE)
(72) Erfinder: MRONCZ, Andreas, 71065 Sindelfingen (DE); TRANCHINA, Gioacchino, 42697 Solingen (DE); FEHLING, Holger, 31535 Neustadt (DE)
(74) Vertreter: Nätebusch, Roderich
(86) Internationale Anmeldenummer: PCT/DE2009/000524
(87) Internationale Veröffentlichungsnummer: WO 2009/127202

(56) Entgegenhaltungen:
- WO-A-01/10308
- US-A- 5 192 284
- US-A- 5 308 327
- US-A- 5 759 187
- US-A1- 2003 100 919
- US-B1- 6 409 733

## Beschreibung

Die vorliegende Erfindung bezieht sich auf einen medizinischen Aufnahmebeutel, der insbesondere mittels einer Schiebeeinrichtung in zusammengelegtem Zustand in einem Arbeitskanal eines Endoskops verschiebbar ist und der nach seinem Entfalten außerhalb des betreffenden Arbeitskanals Gewebeteile und/ oder Fremdkörper aufzunehmen gestattet, mit einem Beutelteil, der eine Beutelteilöffnung und einen diese begrenzenden Randbereich aufweist, welcher mit der Schiebeeinrichtung verbunden ist. Die Erfindung bezieht sich ferner auf ein Verfahren zur Herstellung eines medizinischen Aufnahmebeutels der vorstehend genannten Art.

Medizinische Aufnahmebeutel der vorstehend genannten Art, die auch als Extraktionsbeutel bezeichnet werden, sind in unterschiedlichen Ausgestaltungen bekannt (siehe z.B. DE 42 04 210 A1, DE 44 29 478 C1, DE 196 24 826 C2, US 5.643.283, US 5.906.621, US 6.814.739 B2). Die betreffenden Aufnahmebeutel weisen üblicherweise relativ große Abmessungen auf, und sie sind deshalb in der Regel nicht für einen Einsatz in sehr kleinen Arbeitskanälen von Endoskopen mit Öffnungsweiten von 2mm und darunter geeignet. Darüber hinaus ist die Herstellung der bekannten Aufnahmebeutel, soweit erkennbar, relativ aufwendig. Dies trifft nicht nur für aus Gewebegeflechten hergestellte Aufnahmebeutel zu, sondern auch für aus dichten Materialien, wie Kunststofffolien hergestellte Aufnahmebeutel.

Es ist auch schon ein medizinischer Aufnahmebeutel bekannt (US 2003/100919 A1), der mittels einer Schiebeeinrichtung in zusammengelegtem Zustand in einer Durchgangsöffnung eines Katheters verschiebbar ist und der nach seinem Entfalten außerhalb der betreffenden Durchgangsöffnung Gewebeteile und/oder Fremdkörper aufzunehmen gestattet. Dieser bekannte Aufnahmebeutel weist ein Beutelteil mit einer Beutelöffnung und einem diese begrenzenden Randbereich auf, der mit der Schiebeeinrichtung verbunden ist und der mit einem mit der Schiebeeinrichtung zu verbindenden elastischen Formungselement aus einem Formgedächtnismaterial (Nitinol) versehen ist. Der Beutelteil lässt sich durch Blasformen eines Kunststoffschlauches bilden. Für die Aufnahme des Formungselements aus dem Formgedächtnismaterial ist dabei eine durch Umklappen bzw. Falten und Verkleben oder Verschweißen des Randbereiches des Beutelteiles gebildete Durchgangsöffnung vorgesehen. In dem für die Bildung des Beutelteiles verwendeten Kunststoffschlauch selbst sind indessen keine Öffnungen enthalten.

Es ist außerdem ein medizinischer Aufnahmebeutel bekannt (US 5 759 187 A), der mittels einer Schiebeeinrichtung in zusammengelegtem Zustand in einem Endoskop verschiebbar ist und der nach seinem Entfalten außerhalb des betreffenden Arbeitskanals Gewebeteile und/oder Fremdkörper aufzunehmen gestattet. Dieser bekannte Aufnahmebeutel weist ein Beutelteil mit einer Beutelöffnung und einem diese begrenzenden Randbereich auf, der mit der Schiebeeinrichtung verbunden ist und der mit einem mit der Schiebeeinrichtung zu verbindenden elastischen Formungselement aus einem Formgedächtnismaterial versehen ist. Dieses Formungselement ist bei dem aus einem Netz gebildeten Beutel entweder durch an dessen Öffnungsrand befindliche Schlaufen geführt oder direkt mit dem Beutelrand verbunden. In dem Beutel selbst sind indessen keine Öffnungen für die Aufnahme des Formungselements enthalten.

Es ist ferner ein zur Aufnahme von Gewebeproben dienender medizinischer Aufnahmebeutel bekannt (US 5 192 284 A), der mittels einer Schiebeeinrichtung durch einen Kanal bei einer laparoskopischen Behandlungsprozedur geschoben werden kann. Dieser Aufnahmebeutel enthält einen flexiblen Beutel mit einer in Abstand vom distalen Beutelende aus vorgesehenen ovalförmigen Öffnung für die Aufnahme der Gewebeproben. Im Randbereich dieser Öffnung ist allerdings kein Formungselement vorgesehen.

Es ist überdies ein medizinischer Aufnahmebeutel bekannt (US 6 409 733 B1), der mittels einer Schiebeeinrichtung in zusammengelegtem Zustand im Arbeitskanal eines Endoskops verschiebbar ist und der nach seinem Entfalten außerhalb des betreffenden Arbeitskanals Gewebeteile aufzunehmen gestattet. Dieser Aufnahmebeutel ist in seinem Öffnungsbereich zu einem eine Durchgangsöffnung enthaltenden Randteil umgeklappt, in dessen Durchgangsöffnung ein mit der Schiebeeinrichtung verbundenes Formungselement aufgenommen ist. In dem Aufnahmebeutel selbst sind indessen von Hause aus keine Öffnungen für eine Aufnahme des Formungselements enthalten.

Es ist außerdem eine chirurgische Vorrichtung zur Gewebeentnahme aus einem Körperhohlraum bekannt (WO 01/10308 A). Dazu weist diese Vorrichtung an ihrem in den jeweiligen Körperhohlraum einzuführenden distalen Ende eine Drahtschlaufe aus einem Fonngedächtnismaterial auf, von der ein Beutel aufnehmbar ist. Wie dieser Beutel mit der Drahtschlaufe verbunden ist oder wird, ist allerdings in diesem Zusammenhang nicht näher angegeben.

Es ist schließlich auch schon eine medizinische Vorrichtung, wie ein Abführ- oder Trennbeutel mit einem aufblasbaren Element bekannt (US 5 308 327 A), welches sich von einem zusammengefallenen Zustand in einen aufgeweiteten Zustand ausdehnt, wenn ihm ein Gas oder eine Flüssigkeit unter Druck zugeführt wird. Das betreffende Element umfasst zwar auch einen Aufnahmebeutel, der jedoch an einem mit einer Schiebeeinrichtung verbundenen Haltedraht angeschweißt ist.

Der Erfindung liegt daher die Aufgabe zu Grunde, einen Weg aufzuzeigen, wie ein medizinischer Aufnahmebeutel der eingangs genannten Art auf einfachere Weise als bisher bekannte medizinische Aufnahmebeutel zu gestalten und herzustellen ist, damit er in zusammengefaltetem Zustand durch sehr kleine Öffnungen mit Öff-nungsweiten von etwa 2mm und darunter geschoben werden kann, wie sie insbesondere als Arbeitskanäle in neueren Endoskopen zu finden sind.
Gelöst wird die vorstehende Aufgabe bei einem medizinischen Aufnahmebeutel der eingangs genannten Art erfindungsgemäß dadurch, dass der Beutelteil durch einen aufgeblasenen Bereich eines Kunststoffschlauches gebildet ist, dass der die Beutelteilöffnung begrenzende Randbereich durch einen wenigstens einen Einschnitt aufweisende Schlauchbereich gebildet ist, dass der mit wenigstens einem mit der Schiebeinrichtung zu verbindenden elastischen Formungselement versehen ist, dass die Schiebeeinrichtung im Randbereich des Beutelteiles wenigstens ein Element und vorzugsweise zwei Elemente jeweils aus einer Formgedächtnislegierung enthält, dem bzw. denen eine einer gewünschten Beutelteilöffnung entsprechende Form gegeben ist, und dass das jeweilige Element aus einer Formgedächtnislegierung in einer in dem Kunststoffschlauch enthaltenen Durchgangsöffnung aufgenommen ist.

Die Erfindung bringt den Vorteil mit sich, dass auf besonders einfache Weise ein medizinischer Aufnahmebeutel bereitgestellt werden kann, der in zusammengefaltetem Zustand durch sehr kleine Öffnungen mit Öffnungsweiten von etwa 2mm und darunter geschoben werden kann, so dass er für Feinchirurgieanwendungen geeignet ist. Darüber hinaus wird der Vorteil erzielt, dass der Öffnung des Aufnahmebeutels auf besonders einfache Weise nach dessen Entfalten eine gewünschte Öffnungsform gegeben werden kann und dass das jeweilige Element aus einer Formgedächtnislegierung sicher in dem Aufnahmebeutel untergebracht werden kann.

Zweckmäßigerweise besteht der Kunststoffschlauch aus Polyethylen oder Polyurethan. Dadurch lassen sich besonders einfach zu formende Aufnahmebeutel bereitstellen.

Vorzugsweise ist das jeweilige Element aus einer Formgedächtnislegierung durch einen Nitinoldraht gebildet. Da Nitinoldrähte ohne weiteres erhältlich sind, ergibt sich durch deren Einsatz als Element bzw. Elemente aus einer Formgedächtnislegierung aus Herstellungssicht ein besonders geringer Aufwand.

Zur Herstellung eines medizinischen Aufnahmebeutels gemäß der Erfindung, insbesondere eines mittels einer Schiebeeinrichtung in zusammengelegtem Zustand in einem Arbeitskanal eines Endoskops verschiebbaren Aufnahmebeutels, von dem nach seinem Entfalten außerhalb des betreffenden Arbeitskanals Gewebeteile und/oder Fremdkörper aufnehmbar sind, wird ein Herstellungsverfahren angewandt, bei dem folgende Verfahrensschritte ausgeführt werden:
ein Kunststoffschlauch wird über eine dem Öffnungsbereich des Aufnahmebeutels entsprechende Länge in einer quer zu seiner Längsrichtung verlaufenden Richtung mittels eines Druckfluids zu einem Beutelteil aufgeblasen;
der so aufgeblasene Kunststoffschlauch wird auf der an den genannten Beutelteil angrenzenden unaufgeblasenen Längsseite unter Bildung wenigstens eines Einschnitts in Längsrichtung geschlitzt;
lediglich der Kunststoffschlauch mit dem Beutelteil und mit der mit diesem zusammenhängenden, einen Randbereich des Beutelteils bildenden geschlitzten Längsseite werden von in Schlauchlängsrichtung gegebenenfalls überstehenden Schlauchteilen abgetrennt;
der so gebildete Randbereich wird mit wenigstens einem und vorzugsweise mit zwei die Beutelteilöffnung festlegenden und mit der Schiebeinrichtung zu verbindenden elastischen Formungselementen versehen;
und als jeweiliges elastisches Formungselement wird wenigstens ein Element und vorzugsweise werden zwei Elemente jeweils aus einer Formgedächtnislegierung in eine in dem Randbereich des Kunststoffschlauches gebildete gesonderte Aufnahmeöffnung eingeführt.

Dadurch wird der Vorteil einer besonders einfachen Herstellung eines medizinischen Aufnahmebeutels gemäß der Erfindung erzielt, der in einer derart geringen Größte ausgebildet werden kann, dass er durch sehr kleine Öffnungen mit Öffnungsweiten von etwa 2mm und darunter geschoben werden kann, wie sie insbesondere als Arbeitskanäle in neueren Endoskopen zu finden sind. Dabei kann der Öffnung des Aufnahmebeutels nach dessen Entfalten auf besonders einfache Weise eine gewünschte Öffnungsform gegeben werden.

Vorzugsweise wird der aus Polyethylen oder Polyurethan bestehende Kunststoffschlauch mittels Druckluft aufgeblasen. Dies ermöglicht den Einsatz eines konventionellen Aufblasverfahrens ohne gesonderten Zusatzaufwand.

Das erwähnte Schlitzen des Kunststoffschlauches wird vorzugsweise mechanisch oder unter Einsatz eines Laserstrahles vorgenommen. Somit kann das betreffende Schlitzen besonders einfach erfolgen.

Zweckmäßigerweise wird das Abtrennen von Schlauchteilen, die von dem aufgeblasenen Kunststoffschlauch und von dem diesem quer zur Schlauchlängsrichtung gegenüberliegenden Bereich mit wenigstens einem Einschnitt in Schlauchlängsrichtung überstehen, mechanisch oder unter Einsatz eines Laserstrahles vorgenommen. Dies bringt den Vorteil mit sich, dass sich auf besonders einfache Weise ein vollständiger medizinischer Aufnahmebeutel gemäß der Erfindung herstellen lässt. Vorzugsweise können die Vorgänge des Abtrennens der erwähnten überstehenden Schlauchteile auf mechanischem Wege oder unter Einsatz eines Laserstrahles mit den Vorgängen zum Schlitzen des Kunststoffschlauches so kombiniert werden, dass praktisch nur ein Gesamtbearbeitungsvorgang ausgeführt zu werden braucht.

An Hand von Zeichnungen wird die Erfindung nachstehend beispielhaft näher erläutert.

In den Zeichnungen zeigen
- Fig. 1: eine vergrößerte Endansicht eines für die Herstellung eines medizinischer Aufnahmebeutels gemäß der Erfindung geeigneten Kunststoffschlauches,
- Fig. 2: eine ausschnittweise vergrößerte Seitenansicht des in Fig. 1 in einer vergrößerten Endansicht dargestellten Kunststoffschlauches nach erfolgtem Aufblasen eines Beutelteiles und
- Fig. 3: eine perspektivische Darstellung eines fertig hergestellten medizinischen Aufnahmebeutels gemäß einer Ausführungsform der Erfindung in nicht maßstäblicher Größe.

Bevor auf die Zeichnungen näher eingegangen wird, sei zunächst angemerkt, dass gleiche Elemente in sämtlichen Zeichnungsfiguren durch gleiche Bezugszeichen bezeichnet sind.

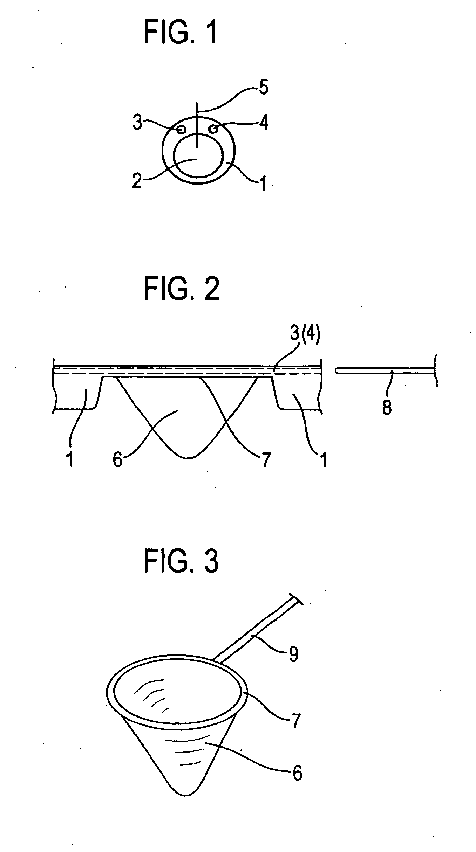

Fig. 1 zeigt in einer vergrößerten Endansicht einen vorzugsweise aus Polyethylen oder Polyurethan bestehenden Kunststoffschlauch 1, der einen hier zylinderförmig ausgebildeten Schlauchinnenraum 2 aufweist, zwischen dem und der Außenwandung des Kunststoffschlauches 1 in dessen oberen Bereich zwei Durchgangsöffnungen 3, 4 enthalten sind. Diese Durchgangsöffnungen 3, 4 sind in dem Material des Kunststoffschlauches 1 bezogen auf eine als Schnittebene 5 eingetragene Mittellinie seitlich versetzt.

Es sei hier darauf hingewiesen, dass in der vergrößerten Endansicht von Fig. 1 der Schlauchinnenraum mit seiner Mitte von der Mitte der kreisförmigen Außenwandung des Kunststoffschlauches 1 versetzt dargestellt ist; es ist jedoch auch ohne weiteres möglich, dass die Mitten des Schlauchinnenraums 2 und der Außenwandung des Kunststoffschlauches 1 zusammenfallen, so dass der Kunststoffschlauch 1 mit seiner Außenwandung konzentrisch zu dem Schlauchinnenraum 2 verläuft.

Der in Fig. 1 in einer vergrößerten Endansicht dargestellte, für die Herstellung eines medizinischen Aufnahmebeutels gemäß der Erfindung geeignete Kunststoffschlauch 1 wird nun, wie dies in der ausschnittweisen vergrößerten Seitenansicht des Kunststoffschlauches 1 in Fig. 2 veranschaulicht ist, über eine bestimmte Länge quer zu seiner Längsrichtung zu einem Beutelteil 6 mittels eines Druckfluids aufgeblasen. Der Beutelteil 6 befindet sich bezogen auf die in Fig. 1 dargestellte Endansicht auf der unteren Seite des dort dargestellten Kunststoffschlauches 1. Der Kunststoffschlauch 1 wird dabei über eine solche Länge zur Bildung des erwähnten Beutelteiles 6 aufgeblasen, die dem Öffnungsbereich des herzustellenden Beutelteiles 6 entspricht. Eine dazu vorgesehene Aufblasvorrichtung wird die Bereiche des Kunststoffschlauches 1 durch Abklemmen abdichten, die den herzustellenden Beutelteil 6 in dem Kunststoffschlauch 1 unmittelbar angrenzend vorgesehen sind. Das Druckfluid, bei dem es sich vorzugsweise um Druckluft handelt, kann dann in den so festgeklemmten Kunststoffschlauch 1 durch eine in Fig. 1 mit 5 bezeichnete Schlitzebene in den zu dem Beutelteil 6 auszubildenden Schlauchinnenraum 2 eingeleitet werden.

Anschließend wird der so aufgeblasene Kunststoffschlauch 1 auf der an den genannten Beutelteil 6 angrenzenden unaufgeblasenen Längsseite - das ist die in Fig. 2. obere Seite des Kunststoffschlauches 1 - unter Bildung wenigstens eines Einschnitts in Längsrichtung entsprechend der in Fig. 1 dargestellten Schlitzebene 5 geschlitzt.

Sodann wird lediglich der Schlauchteil mit dem Beutelteil 6 und mit dem mit diesem zusammenhängenden, einen Randbereich 7 des Beutelteiles 6 bildenden geschlitzten Schlauchlängsteil von in Schlauchlängsrichtung überstehenden Schlauchteilen abgetrennt. Derartige Schlauchteile sind in Fig. 2 jeweils mit 1 bezeichnet.

Sodann wird der so gebildete Randbereich 7 mit wenigstens einem und vorzugsweise mit zwei die Beutelteilöffnung in ihrer Form festlegenden elastischen Formungselementen 8 versehen, die in die im Zusammenhang mit Fig. 1 erwähnten Durchgangsöffnungen 3, 4 eingeführt werden. Als jeweiliges Formungselement 8 wird vorzugsweise ein Element aus einer Formgedächtnislegierung, wie ein Nitinoldraht eingesetzt, dem vor seiner Einführung in die jeweilige Durchgangsöffnung 3 bzw. 4 eine die Beutelteilöffnung festlegende gewünschte Form gegeben wird.

Das Abtrennen der Schlauchteile, die von dem aufgeblasenen Kunststoffschlauch 1 und von dem diesem quer zur Schlauchlängsrichtung gegenüberliegenden Randbereich 7 überstehen - in Fig. 2 jeweils mit 1 bezeichnet - kann mechanisch oder unter Einsatz eines Laserstrahls erfolgen. Auch das Öffnen des Kunststoffschlauches 1 zum Zwecke des Einleitens eines Druckfluids in dessen Schlauchinnenraum 2 und zum Zwecke des Aufschlitzens des Randbereiches 7 kann mechanisch oder unter Einsatz eines Laserstrahls erfolgen. Vorzugsweise lassen sich die erwähnten Vorgänge des Abtrennens und des Schlitzens in einem Gesamtbearbeitungsvorgang kombinieren.

In der perspektivischen Darstellung von Fig. 3 ist ein fertig hergestellter medizinischer Aufnahmebeutel gemäß einer Ausführungsform der Erfindung mit einem trichterförmig ausgebildeten Beutelteil 6 in nicht maßstäblicher Größer veranschaulicht. Dieses trichterförmige Beutelteil 6 ist von dem im Zusammenhang mit Fig. 2 erwähnten Randbereich 7 umgeben, in welchem wenigstens ein, im voniegenden Fall jedoch vorzugsweise zwei Formungselemente 8 enthalten sind. Dabei sind der Randbereich 7 und/oder das in ihm enthaltene jeweilige Formungselement 8 mit einer lediglich schematisch angedeuteten Schiebeeinrichtung 9 verbunden, bei der es sich beispielsweise um eine Schiebestange handeln kann, mit der der medizinische Aufnahmebeutel im zusammengelegten bzw. zusammengefalteten Zustand durch sehr kleine Öffnungen mit Öffnungsweiten von 2mm und darunter, wie durch einen Arbeitskanal eines Arbeitsgerätes, insbesondere eines Endoskops schiebbar ist. Es sei hier angemerkt, dass im zusammengefalteten Zustand des betreffenden medizinischen Aufnahmebeutels dessen Randbereich 7 sich vollständig in Längsrichtung der Schiebeeinrichtung 9 erstrecken wird und dass der Beutelteil 6 um diesen Randbereich 7 herumgewickelt ist. Nach erfolgtem Hindurchschieben durch die erwähnten Öffnungen kann der Beutelteil 6 zur Aufnahme von Gewebeteilen und/oder Fremdkörpern entfaltet werden.

Abschließend sei noch darauf hingewiesen, dass die vorliegende Erfindung nicht auf die aus Fig. 2 und 3 ersichtliche trichterförmige Beutelform beschränkt ist, sondern dass auch durchaus andere Beutelformen, wie beispielsweise quaderförmige oder halbkugelförmige Beutelteile möglich sind. Außerdem kann das Aufblasen des Kunststoffschlauches 1 in Abweichung von der erläuterten Vorgehensweise dadurch erfolgen, dass ein Druckfluid in Längsrichtung des Kunststoffschlauches 1 in dessen Schlauchinnenraum 2 eingeleitet wird. Auch in diesem Fall sind die dem Aufblasbereich 6 benachbarten Schlauchenden abgedrückt, so dass das in den Schlauchinnenraum 2 eingeleitete Druckfluid lediglich den Beutelteil 6 aufzublasen vermag, nicht aber sonst wo aus dem betreffenden Schlauchinnenraum 2 entweichen kann.

### Bezugszeichenliste

- 1: Kunststoffschlauch
- 2: Schlauchinnenraum
- 3: Durchgangsöffnung
- 4: Durchgangsöffnung
- 5: Schlitzebene
- 6: Beutelteil
- 7: Längsseite bzw. Randbereich des Beutelteiles
- 8: Formungselement
- 9: Schiebeeinrichtung

## Patentansprüche

1. Medizinischer Aufnahmebeutel, der insbesondere mittels einer Schiebeeinrichtung (9) in zusammengelegtem Zustand in einem Arbeitskanal eines Endoskops verschiebbar ist und der nach seinem Entfalten außerhalb des betreffenden Arbeitskanals Gewebeteile und/oder Fremdkörper aufzunehmen gestattet,
mit einem Beutelteil (6), der eine Beutelteilöffnung und einen diese begrenzenden Randbereich aufweist, welcher mit der Schiebeeinrichtung verbunden ist,
**dadurch gekennzeichnet, dass** der Beutelteil (6) durch einen aufgeblasenen Bereich (6) eines Kunststoffschlauches (1) gebildet ist,
dass der die Beutelteilöffnung begrenzende Randbereich (7) durch einen wenigstens einen Einschnitt aufweisende Schlauchbereich gebildet ist, der mit wenigstens einem mit der Schiebeinrichtung (9) zu verbindenden elastischen Formungselement (8) versehen ist,
dass die Schiebeeinrichtung (9) im Randbereich des Beutelteiles wenigstens ein Element (8) und vorzugsweise zwei Elemente (8) jeweils aus einer Formgedächtnislegierung enthält, dem bzw. denen eine einer gewünschten Beutelteilöffnung entsprechende Form gegeben ist,
und das jeweilige Element (8) aus einer Formgedächtnislegierung in einer in dem Kunststoffschlauch (1) enthaltenen Durchgangsöffnung (3, 4) aufgenommen ist.

2. Medizinischer Aufnahmebeutel nach Anspruch 1,
**dadurch gekennzeichnet, dass** der Kunststoffschlauch (1) aus Polyethylen oder Polyurethan besteht.

3. Medizinischer Aufnahmebeutel nach Anspruch 1,
**dadurch gekennzeichnet, dass** das jeweilige Element (8) aus einer Formgedächtnislegierung durch einen Nitinoldraht (8) gebildet ist.

4. Verfahren zur Herstellung eines medizinischen Aufnahmebeutels nach einem der Ansprüche 1 bis 3, insbesondere eines mittels einer Schiebeeinrichtung in zusammengelegtem Zustand in einem Arbeitskanal eines Endoskops verschiebbaren Aufnahmebeutels, von dem nach seinem Entfalten außerhalb des betreffenden Arbeitskanals Gewebeteile und/oder Fremdkörper aufnehmbar sind,
**dadurch gekennzeichnet, dass** ein Kunststoffschlauch (1) über eine dem Öffnungsbereich des Aufnahmebeutels entsprechende Länge in einer quer zu seiner Längsrichtung verlaufenden Richtung mittels eines Druckfluids zu einem Beutelteil (6) aufgeblasen wird,
dass der so aufgeblasene Kunststoffschlauch (1) auf der an den genannten Beutelteil (6) angrenzenden unaufgeblasenen Längsseite (7) unter Bildung wenigstens eines Einschnitts (bei 5) in Längsrichtung geschlitzt wird,
dass lediglich der Kunststoffschlauch (1) mit dem Beutelteil (6) und mit der mit diesem zusammenhängenden, einen Randbereich (7) des Beutelteils (6) bildenden geschlitzten Längsseite (7) von in Schlauchlängsrichtung gegebenenfalls überstehenden Schlauchteilen abgetrennt werden,
dass der so gebildete Randbereich (7) mit wenigstens einem und vorzugsweise mit zwei die Beutelteilöffnung festlegenden und mit der Schiebeinrichtung (9) zu verbindenden elastischen Formungselement (8) bzw. Formungselementen (8) versehen wird
und dass als jeweiliges elastisches Formungselement (8) wenigstens ein Element (8) und vorzugsweise zwei Elemente (8) jeweils aus einer Formgedächtnislegierung in eine in dem Randbereich des Kunststoffschlauches (1) gebildete gesonderte Aufnahmeöffnung (3, 4) eingeführt wird bzw. werden.

5. Verfahren nach Anspruch 4, **dadurch gekennzeichnet, dass** der aus Polyethylen oder Polyurethan bestehende Kunststoffschlauch (1) mittels Druckluft aufgeblasen wird.

6. Verfahren nach Anspruch 4 oder 5, **dadurch gekennzeichnet, dass** das Schlitzen (bei 5) des Kunststoffschlauches (1) mechanisch oder unter Einsatz eines Laserstrahles vorgenommen wird.

7. Verfahren nach einem der Ansprüche 4 bis 6, **dadurch gekennzeichnet, dass** das Abtrennen von Schlauchteilen, die von dem aufgeblasenen Kunststoffschlauch (1) und von dem diesem quer zur Schlauchlängsrichtung gegenüberliegenden Bereich mit wenigstens einem Einschnitt (bei 5) in Schlauchlängsrichtung überstehen, mechanisch oder unter Einsatz eines Laserstrahles vorgenommen wird.

## Claims

1. Medical pouch which can be slid in a collapsed state in a working channel of an endoscope, in particular by means of a sliding device (9), and which, after opening up outside the working channel concerned, allows tissue parts and/or foreign bodies to be received, comprising a pouch part (6), which has a pouch part opening and, bounding the latter, a peripheral region which is connected to the sliding device, **characterized in that** the pouch part (6) is formed by an inflated region (6) of a plastic tube (1), **in that** the peripheral region (7) bounding the pouch part opening is formed by a tube region that has at least one incision and is provided with at least one elastic shaping element (8) to be connected to the sliding device (9), **in that** the sliding device (9) includes in the peripheral region of the pouch part at least one element (8), and preferably two elements (8), each of a shape memory alloy, which is or are given a shape corresponding to a desired pouch part opening, and **in that** the respective element (8) of a shape memory alloy is accommodated in a through-opening (3, 4) contained in the plastic tube (1).

2. Medical pouch according to Claim 1, **characterized in that** the plastic tube (1) consists of polyethylene or polyurethane.

3. Medical pouch according to Claim 1, **characterized in that** the respective element (8) of a shape memory alloy is formed by a nitinol wire (8).

4. Method for producing a medical pouch according to one of Claims 1 to 3, in particular a pouch which can be slid in a collapsed state in a working channel of an endoscope, in particular by means of a sliding device, and which, after opening up outside the working channel concerned, allows tissue parts and/or foreign bodies to be received, **characterized in that** a plastic tube (1) is inflated by means of a pressurized fluid over a length corresponding to the opening region of the pouch, in a direction running transversely in relation to its longitudinal direction, to form a pouch part (6), **in that** the plastic tube (1) inflated in this way is slit in the longitudinal direction on the uninflated longitudinal side (7) adjacent to said pouch part (6), thereby forming at least one incision (at 5), **in that** just the plastic tube (1) with the pouch part (6) and with the slit longitudinal side (7) that is joined to and forms a peripheral region (7) of the pouch part (6) are cut off from tube parts possibly projecting in the longitudinal direction of the tube, **in that** the peripheral region (7) formed in this way is provided with at least one elastic shaping element (8), and preferably with two elastic shaping elements (8), fixing the pouch part opening and intended to be connected to the sliding device (9), and **in that** at least one element (8), and preferably two elements (8), in each case of a shape memory alloy is or are inserted as a respective elastic shaping element (8) into a separate receiving opening (3, 4) formed in the peripheral region of the plastic tube (1).

5. Method according to Claim 4, **characterized in that** the plastic tube (1), consisting of polyethylene or polyurethane, is inflated by means of compressed air.

6. Method according to Claim 4 or 5, **characterized in that** the slitting (at 5) of the plastic tube (1) is performed mechanically or by using a laser beam.

7. Method according to one of Claims 4 to 6, **characterized in that** the cutting off of tube parts that project from the inflated plastic tube (1) and from the region opposite it transversely in relation to the longitudinal direction of the tube with at least one incision (at 5) in the longitudinal direction of the tube is performed mechanically or by using a laser beam.

## Revendications

1. Sachet de réception médical qui peut être en particulier déplacé au moyen d'un dispositif de poussée (9) en l'état plié dans un canal de travail d'un endoscope et qui est conçu, une fois déployé, pour recevoir des parties de tissus et/ou des corps étrangers en dehors du canal de travail concerné, comprenant une partie de sachet (6) qui présente une ouverture de partie de sachet et une zone de bordure délimitant celle-ci, laquelle est reliée au dispositif de poussée,
**caractérisé en ce que** la partie de sachet (6) est formée par une partie soufflée (6) d'un tube flexible en plastique (1), que la zone de bordure (7) délimitant l'ouverture de partie de sachet est formée par une section de tube flexible présentant au moins une encoche qui est munie d'au moins un élément de façonnage élastique (8) à relier au dispositif de poussée (9),
que le dispositif de poussée (9) comporte dans la zone de bordure de la partie de sachet au moins un élément (8) et de préférence deux éléments (8), chacun dans un alliage à mémoire de forme, auquel, respectivement auxquels, une forme correspondant à une ouverture de partie de sachet souhaitée est donnée,
et que l'élément (8) respectif dans un alliage à mémoire de forme est reçu dans une ouverture de passage (3, 4) contenue dans le tube flexible en plastique (1).

2. Sachet de réception médical selon la revendication 1,
**caractérisé en ce que** le tube flexible en plastique (1) est en polyéthylène ou en polyuréthane.

3. Sachet de réception médical selon la revendication 1,
**caractérisé en ce que** l'élément (8) respectif dans un alliage à mémoire de forme est formé par un fil de nitinol (8).

4. Procédé destiné à fabriquer un sachet de réception médical selon l'une des revendications 1 à 3, en particulier un sachet de réception qui peut être déplacé au moyen d'un dispositif de poussée en l'état plié dans un canal de travail d'un endoscope, par lequel, une fois déployé, des parties de tissus et/ou des corps étrangers peuvent être reçus en dehors du canal de travail concerné,
**caractérisé en ce qu'**un tube flexible en plastique (1) est soufflé en une partie de sachet (6) au moyen d'un fluide sous pression sur une longueur correspondant à la zone d'ouverture du sachet de réception dans un sens transversal à son sens longitudinal,
que le tube flexible en plastique (1) ainsi soufflé est fendu par formation d'au moins une encoche (sur 5) dans son sens longitudinal sur le côté longitudinal (7) non soufflé adjacent à ladite partie de sachet (6),
que seul le tube flexible en plastique (1), avec la partie de sachet (6) et avec le côté longitudinal (7) fendu formant une zone de bordure (7) de la partie de sachet (6) se rattachant à celui-ci, est séparé de parties de tube flexible dépassant le cas échéant dans le sens longitudinal du tube flexible,
que la zone de bordure (7) ainsi formée est munie d'au moins un et de préférence de deux élément(s) de façonnage (8) élastique(s) à relier au dispositif de poussée (9) et déterminant l'ouverture de partie de sachet,
et que comme élément de façonnage (8) élastique respectif, au moins un élément (8) et de préférence deux éléments (8), chacun dans un alliage à mémoire de forme, est/sont introduit(s) dans une ouverture de passage (3, 4) spéciale formée dans la zone de bordure du tube flexible en plastique (1).

5. Procédé selon la revendication 4,
**caractérisé en ce que** le tube flexible en plastique (1) en polyéthylène ou en polyuréthane est soufflé au moyen d'air comprimé.

6. Procédé selon la revendication 4 ou 5,
**caractérisé en ce que** la fente (sur 5) du tube flexible en plastique (1) est réalisée mécaniquement ou en employant un faisceau laser.

7. Procédé selon l'une des revendications 4 à 6,
**caractérisé en ce que** la séparation de parties du tube flexible, qui dépassent du tube flexible en plastique (1) soufflé et de la section faisant face à celui-ci transversalement au sens longitudinal du tube flexible avec au moins une encoche (sur 5) dans le sens longitudinal du tube flexible, s'effectue mécaniquement ou en employant un faisceau laser.
